(19) Europäisches Patentamt
European Patent Office
Office européen des brevets

(11) Veröffentlichungsnummer: **0 107 623**
**A1**

(12) # EUROPÄISCHE PATENTANMELDUNG

(21) Anmeldenummer: **83810480.0**

(22) Anmeldetag: **17.10.83**

(51) Int. Cl.³: **C 07 D 317/46,** C 07 D 405/06, A 61 K 31/335, A 61 K 31/44

(30) Priorität: **22.10.82 CH 6164/82**

(43) Veröffentlichungstag der Anmeldung: **02.05.84 Patentblatt 84/18**

(84) Benannte Vertragsstaaten: **AT BE CH DE FR GB IT LI LU NL SE**

(71) Anmelder: **CIBA-GEIGY AG, Postfach, CH-4002 Basel (CH)**

(72) Erfinder: **Habicht, Ernst, Dr., Bienenstrasse 13, CH-4104 Oberwil (CH)**
Erfinder: **Zbinden, Paul, Im Rainacker 6, CH-4108 Witterswil (CH)**

(54) **Neue Benzodioxolderivate, Verfahren zu deren Herstellung und diese enthaltende pharmazeutische Zusammensetzungen.**

(57) Die Erfindung betrifft neue Benzodioxolderivate der allgemeinen Formel I

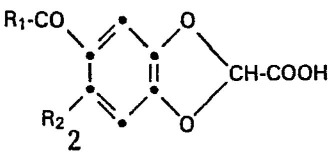

(I)

in welcher $R_1$ Pyridyl, Phenyl, 3-Halogenphenyl oder Phenyl, das in 2- und/oder 6-Stellung unabhängig voneinander durch Niederalkyl oder Halogen (di)substituiert ist, bedeutet, und $R_2$ Niederalkyl oder Halogen ist, Salze von Verbindungen der allgemeinen Formel I mit Basen, sowie Säureadditionssalze von Verbindungen der allgemeinen Formel I, deren Rest $R_1$ basischen Charakter aufweist, sowie Verfahren zur Herstellung der obigen Verbindungen und ihrer Salze und diese enthaltende pharmazeutische Zusammensetzungen. Diese neuen Stoffe besitzen diuretische und zusätzlich urikosurische Wirksamkeit und können, vorzugsweise in Form entsprechender pharmazeutischer Zusammensetzungen, zur Behandlung von Oedemen und der Hypertension Verwendung finden.

EP 0 107 623 A1

CIBA-GEIGY AG 4-14162/13375/-

Basel (Schweiz)

Neue Benzodioxolderivate, Verfahren zu deren Herstellung und diese enthaltende pharmazeutische Zusammensetzungen.

Die Erfindung betrifft neue Benzodioxolderivate, Verfahren zu deren Herstellung und diese enthaltende pharmazeutische Zusammensetzungen, sowie die Verwendung dieser neuen Stoffe und pharmazeutischen Zusammensetzungen.

Die erfindungsgemässen neuen Verbindungen entsprechen der allgemeinen Formel I

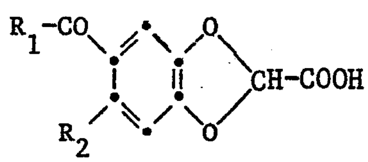

(I) ,

in welcher $R_1$ Pyridyl, Phenyl, 3-Halogenphenyl oder Phenyl, das in 2- und/oder 6-Stellung unabhängig voneinander durch Niederalkyl oder Halogen (di)substituiert ist, bedeutet, und $R_2$ Niederalkyl oder Halogen ist. Sie können als Racemate oder optische Antipoden, oder bei entsprechender Bedeutung der Variabeln auch als Racematgemisch vorliegen. Ebenfalls Gegenstand der Erfindung sind Salze von Verbindungen der allgemeinen Formel I mit Basen, sowie Säureadditionssalze von Verbindungen der allgemeinen Formel I, deren Rest $R_1$ basischen Charakter aufweist, sowie die Herstellung solcher Salze. Soweit nicht anderes bemerkt, werden vor- und nachstehend unter niederen Resten oder Verbindungen solche mit höchstens 7, vorzugsweise höchstens 4 Kohlenstoffatomen verstanden.

Pyridyl ist z.B. 2- oder 4-Pyridyl und insbesondere 3-Pyridyl.

Halogen als Substituent von Phenyl in $R_1$ ist Fluor, Jod, und insbesondere Chlor und Brom.

Halogen als $R_2$ ist Brom, Jod, insbesondere Fluor oder vor allem Chlor.

Niederalkyl ist z.B. Aethyl, Propyl, Isopropyl, Butyl, sek. Butyl, Isobutyl oder tert.Butyl und insbesondere Methyl.

Salze der neuen Verbindungen sind insbesondere Salze mit Basen, vor allem pharmazeutisch verwendbare Salze solcher Verbindungen mit Basen. Als solche Salze mit Basen kommen beispielsweise Alkalimetall- oder Erdalkalimetallsalze, wie Natrium-, Kalium-, Calcium- oder Magnesiumsalze, ferner Ammoniumsalze mit Ammoniak oder organischen Aminen, wie Mono- oder Diniederalkylaminen, z.B. Methylamin, Aethylamin, Dimethylamin oder Diäthylamin, oder Mono-, Di- oder Tri(hydroxyalkyl)-aminen, z.B. 2-Aminoäthanol, 2,2'-Iminodiäthanol oder 2,2',2"-Nitrilotriäthanol, oder Tri(hydroxyalkyl)-alkylaminen, z.B. 2-Amino-2,2-bis-(hydroxymethyl)-äthanol, in Betracht.

Als Säureadditonssalze, insbesondere pharmazeutisch annehmbare Säureadditionssalze von Verbindungen der allgemeinen Formel I, in denen $R_1$ basischen Charakter aufweist, kommen beispielsweise solche mit geeigneten anorganischen Säuren, wie Halogenwasserstoffsäuren, z.B. Chlorwasserstoffsäure oder Bromwasserstoffsäure, ferner Salpetersäure, Schwefelsäure oder Phosphorsäure, oder mit geeigneten organischen Säuren, wie Carbonsäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, oder organischen Sulfonsäuren, wie gegebenenfalls Hydroxy enthaltenden Niederalkansulfonsäuren, z.B. Methansulfonsäure, Aethansulfonsäure, 2-Hydroxyäthansulfonsäure oder Aethan-1,2-disulfonsäure, oder Arylsulfonsäuren, z.B. Benzolsulfonsäure, 4-Methyl-benzolsulfonsäure oder Naphthalin-2-sulfonsäure, oder weiteren sauren Substanzen, wie Ascorbinsäure, in Frage.

Die neuen Verbindungen der allgemeinen Formel I und ihre Salze besitzen wertvolle pharmakologische Eigenschaften. Sie wirken insbesondere diuretisch und natriuretisch an der Ratte im Dosisbereich von 10 bis 1000 mg/kg per os und am Hund in Dosen ab 20 mg/kg per os, wie durch Sammeln des Harns während 3 Stunden nach Verabreichung (Ratte) bzw. stündlich während 5 Stunden nach Verabreichung (Hund) und Bestimmung des Harnvolumens und der Natrium-, Kalium- und Chlorionen festgestellt werden kann. Hierbei wird die Kalium-Ausscheidung weniger stark gesteigert als die Natrium-Ausscheidung; hervorzuheben ist auch die gute Verträglichkeit.

Weiter besitzen die Verbindungen der allgemeinen Formel I uricosurische Wirksamkeit, wie sich z.B. anhand von Versuchen am Cebus-Affen (Cebus apella) zeigen lässt. Bei diesen Versuchen wird den Versuchstieren in Pentobarbital-Narkose Polyfructosan in Ringer-Lösung intravenös infundiert und die Prüfsubstanz als wässrige Lösung in ansteigenden Dosen intravenös injiziert. Vor der ersten Prüfsubstanz-Verabreichung und nach jeder Prüfsubstanzdosis wird während je drei 10-minütigen Perioden der Harn gesammelt und vor der ersten und nach der letzten Sammelperiode jeweils arteriell Blut entnommen. Die Harnsäure- und Polyfructosan-clearance wird aus deren Plasma- und Urin-Konzentration berechnet und schliesslich die fraktionelle Hanrsäureausscheidung (Fractional excretion of uric acid, $FE_{UR}$) als Quotient von Harnsäure-clearance und glomerulärer Filtrationsrate bestimmt. Verbindungen der allgemeinen Formel I zeigen in diesem

Test Wirkungen im Dosisbereich von 1 bis 10 mg/kg i.v. Entsprechend können die Verbindungen der allgemeinen Formel I und ihre pharmazeutisch annehmbaren Salze als kaliumschonende Diuretika mit urikusorischer Zusatzwirkung, z.B. zur Behandlung von Oedemen und der Hypertension, verwendet werden.

Die Erfindung betrifft ganz besonders Verbindungen der allgemeinen Formel I, in denen $R_1$ 3-Pyridyl, Phenyl, 3-Halogenphenyl, insbesondere 3-Chlorphenyl, oder Phenyl, das in 2- und/oder 6-Stellung unabhängig voneinander durch Niederalkyl, insbesondere Methyl, oder Halogen, insbesondere Chlor oder Brom, (di)substituiert ist, bedeutet und $R_2$ Niederalkyl, insbesondere Methyl oder Aethyl, oder Halogen, insbesondere Chlor oder Brom bedeutet, sowie Salze von solchen Verbindungen mit Basen, sowie Säureadditionssalze von solchen Verbindungen der allgemeinen Formel I, deren Rest $R_1$ basischen Charakter aufweist.

Die neuen Verbindungen der allgemeinen Formel I und ihre Salze werden in an sich bekannter Weise hergestellt, indem man

a) eine Verbindung der allgemeinen Formel II

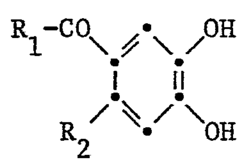

(II) ,

in welcher $R_1$ und $R_2$ die unter der Formel I angegebene Bedeutung haben, oder ein Salz davon mit einer Verbindung der allgemeinen Formel III

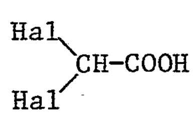

(III) ,

in welcher Hal Halogen bedeutet, oder einem Salz einer solchen Verbindung umsetzt, oder

b) in einer Verbindung der allgemeinen Formel IV

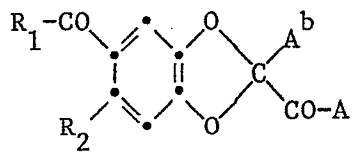

(IV),

in welcher $A^b$ Carboxy, Niederalkoxycarbonyl oder Acetyl bedeutet, A Hydroxy ist, und $R_1$ und $R_2$ die unter der Formel I angegebene Bedeutung haben, den Rest $A^b$ durch Wasserstoff ersetzt, oder

c) zur Herstellung einer Verbindung der allgemeinen Formel I, in welcher $R_1$ und $R_2$ die unter der Formel I angegebene Bedeutung haben, oder eines Salzes dieser Verbindung, in einer Verbindung der allgemeinen Formel VII

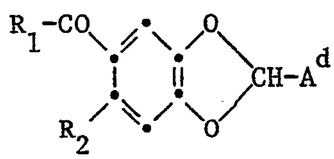

(VII) ,

in welcher $A^d$ eine in die Carboxygruppe überführbare Gruppe bedeutet, und $R_1$ und $R_2$ die unter der Formel I angegebene Bedeutung haben, die Gruppe $A^d$ in die Carboxygruppe in freier oder Salzform überführt, und gewünschtenfalls eine erhaltene Verbindung der allgemeinen Formel I in an sich bekannter Weise in eine andere Verbindung der allgemeinen Formel I überführt, und/oder eine als Racemat erhaltene Verbindung der allgemeinen Formel I in die optischen Antipoden zerlegt, und/oder eine erhaltene Verbindung der allgemeinen Formel I in ein Salz mit einer Base überführt oder eine solche Verbindung aus einem erhaltenen Salz freisetzt, oder eine erhaltene Verbindung der allgemeinen Formel I von basischem Charakter in ein Säureadditionssalz überführt oder eine solche Verbindung aus einem erhaltenen Salz freisetzt.

In den Ausgangsstoffen der allgemeinen Formel III ist Hal vorzugsweise Chlor oder Brom, kann aber auch Fluor oder Jod sein, wobei

auch zwei unter sich verschiedene Halogenatome vorliegen können. Die Umsetzungen gemäss Verfahren a) werden vorzugsweise in unter den Reaktionsbedingungen inerten organischen Lösungsmitteln, beispielsweise in ätherartigen, wie z.B. Dibutyläther, 1,2-Dimethoxyäthan, Diäthylenglykoldimethyläther, Tetrahydrofuran oder Dioxan; alkoholartigen, wie z.B. Methanol, Aethanol, Isopropanol, Butanol, 2-Methox]-äthanol oder 2-Aethoxyäthanol; oder amidartigen, wie z.B. Dimethylformamid oder N,N,N',N',N",N"-Hexamethylphosphorsäuretriamid; oder in Kohlenwasserstoffen, wie z.B. Petroläther, Cyclohexan, Benzol oder Toluol durchgeführt. Umsetzungen mit freien Verbindungen der allgemeinen Formel II wie auch mit freien Halogenessigsäuren der allgemeinen Formel III werden vorzugsweise in Gegenwart von basischen Stoffen durchgeführt. Als solche können beispielsweise organische oder anorganische Derivate von Alkali- oder Erdalkalimetallen, als organische Derivate z.B. Alkalimetall- oder Erdalkalimetallalkoxide, wie Natrium- oder Lithiummethoxid, -äthoxid, -n-butoxid oder -tert.-butoxid, bzw. Barium-methoxid, oder als anorganische Derivate z.B. entsprechende Hydroxide, wie Natrium-, Kalium- oder Calciumhydroxid, oder Carbonate, wie z.B. Natrium- oder Kaliumcarbonat, verwendet werden. Insbesondere Carbonate können in grösserem, z.B. bis fünffachem Ueberschuss eingesetzt werden. Bei Verwendung von Carbonaten können auch noch weitere organische Lösungsmittel, wie Niederalkanone, z.B. Aceton oder 2-Butanon, als genügend inert in Betracht kommen.

Als Salze von Verbindungen der allgemeinen Formel II und von gegebenenfalls verwendeten, unter die allgemeine Formel III fallenden Dihalogenessigsäuren eignen sich beispielsweise entsprechende Alkalimetallsalze oder Erdalkalimetallsalze. Die Reaktionstemperaturen liegen beispielsweise zwischen Raumtemperatur und etwa 150°C und vorzugsweise zwischen etwa 70 und 120°C.

Eine Anzahl Ausgangsstoffe der allgemeinen Formeln II und III sind bekannt und weitere analog zu den bekannten Verbindungen herstellbar. So kann man beispielsweise Ausgangsstoffe der allgemeinen Formel II herstellen, indem man zunächst Veratrol, das entsprechend der Definition für $R_2$ substituiert sein kann, mit einem von einer Carbonsäure der Formel $R_1$-COOH abgeleiteten Acylhalogenid nach Friedel-Crafts, z.B. mittels Aluminiumchlorid in 1,2-Dichloräthan bei Raumtemperatur, zum entsprechenden Keton kondensiert und in diesem die beiden Methoxygruppen in an sich bekannter Weise, z.B. durch Erhitzen mit Pyridin-hydrochlorid oder mit 48%-iger Bromwasserstoffsäure in Essigsäure, spaltet.

Zur Durchführung des Verfahrens b) erhitzt man beispielsweise einen Ausgangsstoff der allgemeinen Formel IV, in welchem $A^b$ Carboxy bedeutet, A Hydroxy ist und $R_1$ und $R_2$ die unter der Formel I angegebene Bedeutung haben, in An- oder Abwesenheit eines Katalysators, z.B. Kupferpulver, und/oder eines Lösungs- oder Verdünnungsmittels, wie z.B. o-Dichlorbenzol oder 1,2,3,4-Tetrahydronaphthalin, bis die mindestens annähernd äquimolare Menge Kohlendioxid freigesetzt ist. Ausgangsstoffe der allgemeinen Formel IV, in denen $A^b$ Carboxy und A Hydroxy bedeutet, werden beispielsweise durch Hydrolyse von entsprechenden Verbindungen, in denen A $OR_4$ und darin $R_4$ Niederalkyl ist und als bzw. anstelle von $A^b$ Niederalkoxycarbonyl oder Cyano steht, in saurem oder alkalischem Medium, beispielsweise durch Erwärmen mit einer starken Mineralsäure in wässrigem oder wässrig-organischem, z.B. wässrig-niederalkanolischem Medium, bzw. mit der mindestens doppeltmolaren Menge eines Alkalimetallhydroxids, insbesondere Natrium- oder Kaliumhydroxid, z.B. in einem Niederalkanol, wie Methanol, Aethanol, Isopropanol oder n-Butanol, oder in einem Niederalkandiol oder Monoalkyläther desselben, z.B. Aethylenglykol, 2-Methoxyäthanol oder 2-Aethoxyäthanol, hergestellt, wobei den genannten Lösungsmitteln gegebenenfalls Wasser im

Volumenverhältnis von Lösungsmittel zu Wasser von ca. 10:1 bis 1:2 zugefügt wird. Ferner kann als Reaktionsmedium auch Wasser oder z.B. ein Gemisch von Wasser mit wasserlöslichen, ätherartigen Lösungsmitteln, wie Dioxan oder Tetrahydrofuran, verwendet werden.

Erfolgt die Hydrolyse in einer wasserhaltigen Mineralsäure, so kann die verfahrensgemässe Decarboxylierung daran schliessend, d.h. im gleichen Medium und Arbeitsgang durchgeführt werden.

Die Ausgangsstoffe der allgemeinen Formel IV, in denen $A^b$ Niederalkoxycarbonyl oder Acetyl ist, sowie die weiter oben genannten Vorprodukte zu Verbindungen der allgemeinen Formel IV mit Carboxy als Rest $A^b$, die als bzw. anstelle von $A^b$ Niederalkoxycarbonyl bzw. Cyano enthalten, können analog Verfahren a) durch Umsetzung von Verbindungen der allgemeinen Formel II mit geminalen Dihalogenverbindungen, die sich von solchen der allgemeinen Formel III durch das Vorliegen von Niederalkoxycarbonyl, Acetyl oder Cyano anstelle des neben beiden Halogenatomen befindlichen Wasserstoffatoms unterscheiden, in Gegenwart einer Base hergestellt werden.

Bei der Herstellung von Verbindungen der allgemeinen Formel I gemäss Verfahren c) kann die Umwandlung einer Gruppe $A^d$ in die Carboxygruppe in an sich bekannter Weise, insbesondere durch Hydrolyse in alkalischem oder saurem Medium erfolgen, wobei man im ersteren Fall auch direkt ein Salz erhalten kann. Ausgangsstoffe für die Hydrolyse sind zunächst Verbindungen der allgemeinen Formel VII, besonders solche, die eine leicht hydrolysierbare Gruppe als $A^d$ tragen, wie z.B. einen Niederalkylester, im weiteren aber auch andere funktionelle Derivate der als Endstoffe gewünschten Carbonsäuren, wie z.B. Nitrile und Imidoester, insbesondere Imidoniederalkylester, von unter die allgemeine Formel I fallenden Carbonsäuren. Die Hydrolyse erfolgt beispielsweise in niederalkanolischen oder wässrig-niederalkanolischen

BAD ORIGINAL

Alkalihydroxidlösungen bei Raumtemperatur bis etwa 100°C bzw. Siedetemperatur des Reaktionsmediums. Niederalkylester, wie Methyl- oder Aethylester und andere leicht spaltbare Ester von unter die allgemeine Formel I fallenden Carbonsäuren können unter noch milderen Bedingungen, z.B. in Gegenwart von Kalium- oder Natriumcarbonat bei Raumtemperatur oder nötigenfalls schwach erhöhten Temperaturen von z.B. 40°C, in wässerig-organischem Medium, z.B. in einem mit Wasser mischbaren Lösungsmittel, wie z.B. 1,2-Dimethoxyäthan, mit Wasser hydrolysiert werden. Aus den dabei zunächst erhaltenen Alkalimetallsalzlösungen der unter die allgemeine Formel I fallenden Carbonsäuren kann man entweder durch Einengen und Abfiltrieren bzw. Eindampfen und Umkristallisieren direkt das entsprechende reine Alkalisalz gewinnen oder zunächst die Carbonsäure freisetzen, anschliessend z.B. durch Umkristallisation reinigen und gewünschtenfalls wiederum in ein Salz mit einer geeigneten anorganischen oder organischen Base überführen. Funktionelle Derivate der unter die allgemeine Formel I fallenden Carbonsäuren lassen sich ferner auch in saurem Medium, z.B. durch Erwärmen in einer mit Wasser verdünnten, z.B. 60-70%igen Schwefelsäure oder in niederalkanolisch-wässeriger Salzsäure, in die freie Carbonsäure der allgemeinen Formel I überführen.

Die benötigten funktionellen Derivate von Carbonsäuren, wie z.B. Nitrile, welche unter die allgemeine Formel VII fallen, werden z.B. analog Verfahren a) oder b) hergestellt.

Zur Herstellung einer Verbindung der allgemeinen Formel VII kann man ferner eine Verbindung der allgemeinen Formel V

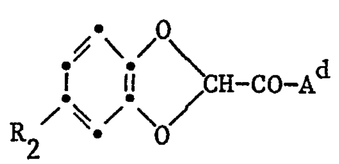

(V)

mit einem Anhydrid einer Verbindung der allgemeinen Formel VI

$$R_1-COOH \qquad (VI) ,$$

worin $A^d$ die unter der Formel VII angegebene Bedeutung hat und $R_2$ und $R_1$ die unter der Formel I angegebene Bedeutung haben, umsetzen.

Für dieses Verfahren verwendet man als Anhydride von Verbindungen der allgemeinen Formel VI beispielsweise deren Halogenide, wie Chloride oder Bromide, ferner z.B. deren symmetrische Anhydride. Geeignete Katalysatoren für die Umsetzung sind z.B. solche üblicher Friedel-Crafts-Kondensationen,wie Aluminiumchlorid oder Zinn(IV)-chlorid, ferner z.B. Zinkchlorid, weiter konz. Schwefelsäure, Phosphorsäure, Polyphosphorsäure oder Pyrophosphorsäure. Die vorgenannten Säuren werden bevorzugt verwendet, wenn man als Derivat einer Carbonsäure der allgemeinen Formel VI ein symmetrisches Carbonsäureanhydrid einsetzt. Die Umsetzung wird vorzugsweise in einem Lösungsmittel vorgenommen. Als solche kann man beispielsweise Halogenkohlenwasserstoffe, wie 1,2-Dichloräthan, Tetrachlorkohlenstoff, Methylenchlorid, o-Dichlorbenzol, weiter z.B. aliphatische oder cycloaliphatische Kohlenwasserstoffe, wie Heptan oder Cyclohexan, Nitrokohlenwasserstoffe, wie Nitromethan, Nitrocyclohexan oder Nitrobenzol und ferner unter milden Bedingungen auch Schwefelkohlenstoff verwenden. Die Reaktionstemperatur liegt zwischen etwa -20° und +80°C, vorzugsweise zwischen etwa 0° und Raumtemperatur.

Die Ausgangsstoffe der allgemeinen Formel V können ihrerseits analog Verfahren a) aus gegebenenfalls entsprechend der Definition für $R_2$ substituiertem Brenzcatechin, wie z.B. Homobrenzcatechin (4-Methylbrenzcatechin),und funktionellen Derivaten von Dihalogenessigsäuren der allgemeinen Formel III hergestellt werden.
Von den als zweite Reaktionskomponenten benötigten funktionellen Derivaten von Verbindungen der allgemeinen Formel VI sind manche bekannt und weitere analog zu den bekannten herstellbar.

Erhaltene salzbildende Verbindungen der Formel I können in an sich bekannter Weise in Salze übergeführt werden, z.B. mit entsprechenden Basen, wie z.B. Alkalimetallhydroxiden, in Salze mit Basen, oder solche von basischem Charakter in ihre Säureadditionssalze. Vorzugsweise werden pharmazeutisch annehmbare Salze hergestellt.

Erhaltene Salze können in an sich bekannter Weise in die freien Verbindungen umgewandelt werden, z.B. durch Behandeln mit einem sauren Reagens, wie einer Mineralsäure, bzw. mit einer Base, z.B. einer Alkalimetallhydroxidlösung, wie Natronlauge.

Die Verbindungen einschliesslich ihrer Salze können auch in Form ihrer Hydrate erhalten werden, oder ihre Kristalle können das zur Kristallisation verwendete Lösungsmittel einschliessen.

Infolge der engen Beziehung zwischen den neuen Verbindungen der allgemeinen Formel I in freier Form und in Form ihrer Salze mit Basen, sowie von solchen Verbindungen, deren Rest $R_1$ basischen Charakter aufweist, in freier Form oder in Form von Säureadditionssalzen, sind im vorausgegangenen und nachfolgend unter den freien Verbindungen oder ihren Salzen sinngemäss gegebenenfalls auch die entsprechenden Salze bzw. freien Verbindungen zu verstehen.

Die neuen Verbindungen können, je nach der Anzahl der Asymmetriezentren sowie auch nach der Wahl der Ausgangsstoffe und Arbeitsweisen, in Form von Racematen oder Racematgemischen (Diastereomerengemischen) oder gegebenenfalls auch als reine Antipoden erhalten werden.

Erhaltene Racematgemische können auf Grund der physikalisch-chemischen Unterschiede der Bestandteile in bekannter Weise in die reinen Racemate bzw. Diastereomeren aufgetrennt werden, beispielsweise durch

Chromatographie und/oder fraktionierte Kristallisation. Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung eines sauren Endstoffes der allgemeinen Formel I mit einer mit der racemischen Säure Salze bildenden optisch aktiven Base, bzw. Umsetzung eines basischen Endstoffes der allgemeinen Formel I mit einer optisch aktiven Säure, und Trennung der auf diese Weise erhaltenen Salze, z.B. auf Grund ihrer verschiedenen Löslichkeiten, in die Diastereomeren, aus denen die Antipoden durch Einwirkung geeigneter Mittel freigesetzt werden können. Vorteilhaft isoliert man den wirksameren der beiden Antipoden.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes und/oder Racemates bzw. Antipoden verwendet oder insbesondere unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen. Neue Ausgangsstoffe und Verfahren zu ihrer Herstellung bilden ebenfalls einen Gegenstand der vorliegenden Erfindung.

Die Erfindung betrifft im weiteren pharmazeutische Präparate, die Verbindungen der allgemeinen Formel I als Wirkstoffe enthalten, sowie Verfahren zu ihrer Herstellung.

Bei den erfindungsgemässen pharmazeutischen Präparaten handelt es sich um solche zur enteralen, wie oralen oder rektalen, sowie zur parenteralen Verabreichung an Warmblüter. Die Dosierung des Wirkstoffs, der alleine oder zusammen mit dem üblichen Träger- und Hilfsmaterial appliziert wird, hängt von der Warmblüterspezies, deren Alter und dem individuellen Zustand sowie der Applikationsweise ab. Die täglichen Dosen bewegen sich zwischen 0,5 und 30 mg/kg für Mammalien und liegen für solche von etwa 70 kg Gewicht je nach individuellem Zustand und Alter vorzugsweise zwischen 25 und 900 mg, insbesondere zwischen 50 und 600 mg. Entsprechende orale Doseneinheitsformen, z.B. Dragées oder Tabletten oder Kapseln, enthalten vorzugsweise 12,5 bis 300 mg, insbesondere 25 bis 200 mg eines erfingungsgemässen Wirkstoffes, d.h. einer Verbindung der allgemeinen Formel I oder eines pharmazeutisch annehmbaren Salzes einer zur Salzbildung befähigten Verbindung der allgemeinen Formel I, zusammen mit pharmazeutischen Trägerstoffen.

Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister und Verwendung z.B. von Mais-, Weizen-, Reis- oder Kartoffelstärke, Gelatine, Traganth, Methylcellulose und/oder Polyvinylpyrrolidon, und/oder,

wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten. Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere, oral anwendbare pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffes mit einer Grundmasse enthalten; als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffes in wasserlöslicher Form, z.B. eines wasserlöslichen Salzes, ferner Suspensionen des Wirkstoffes, wie entsprechende ölige Injektionssuspensionen, wobei man geeignete lipophile Lösungsmittel oder Vehikel, wie fette Oele, z.B. Sesamöl, oder synthetische Fettsäureeester, z.B. Aethyloleat oder Triglyceride, verwendet, oder wässrige Injektionssuspensionen, welche viskositätserhöhende Stoffe, z.B. Natriumcarboxymethylcellulose, Sorbit und/oder Dextran und gegebenenfalls Stabilisatoren enthalten.

Die Erfindung betrifft ebenfalls die Verwendung der neuen Verbindungen der Formel I und der pharmazeutisch annehmbaren Salze von solchen als pharmakologisch aktive Verbindungen, insbesondere als Diuretika mit urikosurischer Zusatzwirkung, vorzugsweise in Form von pharmazeutischen Präparaten in einem Verfahren zur prophylaktischen und/oder therapeutischen Behandlung des tierischen oder menschlichen Körpers, insbesondere zur Behandlung von Oedemen und/oder der Hypertension.

Die nachfolgenden Beispiele illustrieren die oben beschriebene Erfindung; sie sollen jedoch diese in ihrem Umfang in keiner Weise einschränken. Temperaturen werden in Celsiusgraden angegeben.

Beispiel 1: Zu einer Lösung von 31,2 g (0,15 Mol) 5-Methyl-1,3-benzodioxol-2-carbonsäure-äthylester und 42 g 2-Chlorbenzoylchlorid in 350 ml 1,2-Dichloräthan werden bei 5° bis 10° unter Rühren 46,6 g (0,35 Mol) Aluminiumchlorid innerhalb 25 Minuten portionenweise zugegeben. Hierauf wird das Reaktionsgemisch noch 90 Minuten bei 5-10° und 30 Minuten bei Raumtemperatur gerührt, worauf alles Aluminiumchlorid aufgelöst ist. Nach ca. 24 Stunden Stehenlassen bei Raumtemperatur wird das Reaktionsgemisch auf ca. 2 kg Eis-Wasser-Gemisch gegossen. Die organische Phase wird abgetrennt, einmal mit 2-n.Salzsäure, zweimal mit Wasser, einmal mit 1-n.Natriumbicarbonatlösung und noch zweimal mit Wasser gewaschen, dann über Natriumsulfat getrocknet und im Vakuum eingedampft. Der als Rückstand erhaltene 5-(2-Chlorbenzoyl)-6-methyl-1,3-benzodioxol-2-carbonsäure-äthylester fällt als Oel an, das direkt weiterverarbeitet wird.

34 g (0,1 Mol) des obigen Aethylesters werden in 180 ml Aethanol gelöst und bei Raumtemperatur 87 ml 2-n.Natronlauge zugegeben, wobei Dunkelfärbung und leichte Erwärmung eintritt. Nach 2 Stunden Stehenlassen bei Raumtemperatur wird das Aethanol im Vakuum abdestilliert, der Rückstand mit ca. dem halben Volumen Wasser versetzt und zweimal mit Aether extrahiert. Hierauf wird die wässrige Lösung mit ca. 35 ml 6-n.Salzsäure angesäuert und die ausgeschiedene Säure mit Aethylacetat extrahiert. Die organische Phase wird zweimal mit Wasser gewaschen, getrocknet und eingedampft, wobei die rohe 5-(2-Chlorbenzoyl)-6-methyl-1,3-benzodioxol-2-carbonsäure als sandfarbenes Pulver zurückbleibt. Zur Reinigung wird sie in wenig Aethylacetat gelöst, das gleiche Volumen Cyclohexan zugegeben, dann die heisse Lösung mit Bleicherde versetzt und durch Diatomeenerde filtriert. Das Filtrat wird im Rotationsverdampfer eingeengt, bei Raumtemperatur stehengelassen und schliesslich auf ca. 10° gekühlt. Die Kristalle werden abfiltriert, mit wenig Aethylacetat-Cyclohexan gewaschen und nochmals aus Aethylacetat-Cyclohexan umkristallisiert. Die so erhaltene Substanz schmilzt bei 133-135°.

Beispiel 2: Ein Gemisch von 25,9 g (0,084 Mol (2-Bromphenyl)-(3,4-dihydroxy-6-methylphenyl)-methanon, 68 g Kaliumcarbonat, wasserfrei, und 21,3 g (0,0975 Mol) Dibromessigsäure wird in 150 ml Dimethylformamid 5 Stunden bei 80° unter Stickstoffschutz gerührt. Nun wird das Dimethylformamid im Rotationsverdampfer so weit als möglich abdestilliert. Der Rückstand wird in 300 ml Wasser gelöst und in der Kälte mit verdünnter Salzsäure kongosauer gestellt. Die ausfallende rohe 5-(2-Brombenzoyl)-6-methyl-1,3-benzodioxol-2-carbonsäure wird mit Aethylacetat extrahiert und anschliessend aus Toluol umkristallisiert; Smp. 123-125°.

Das Ausgangsketon wird auf folgende Weise hergestellt:

a) 20,7 g (0,1 Mol) 2-Brombenzoesäure und 15,2 g (0,1 Mol) 1,2-Dimethoxy-4-methylbenzol werden mit einer Lösung von 20 g Biphosphorpentoxid in 200 ml Methansulfonsäure übergossen und die Mischung unter Rühren 30 Minuten auf 70° erhitzt. Hierauf giesst man das Gemisch auf Eiswasser und extrahiert mit Aether. Die Aetherextrakte ergeben nach dem Eindampfen das (2-Bromphenyl)-(3,4-dimethoxy-6-methylphenyl)-methanon als braunes Produkt von honigartiger Konsistenz.

b) 38,8 g 4-(2-Brombenzoyl)-5-methyl-1,2-dimethoxybenzol und 80 g Pyridin-Hydrochlorid werden unter Rühren mit einem Magnetrührer 2 Stunden auf 180-200° erhitzt. Nach Abkühlen der erhaltenen Schmelze wird sie mit 1000 ml 1-n.Salzsäure versetzt und mit Aether extrahiert. Aus den getrockneten und eingedampften Aetherextrakten erhält man nach Kristallisation aus Toluol das (2-Bromphenyl)-(3,4-dihydroxy-6-methylphenyl)-methanon als farblose Kristalle vom Smp. 177-178°.

Beispiel 3: Analog Beispiel 2 erhält man unter Verwendung von 13,0 g (0,0435 Mol) 4-(2,6-Dichlorbenzoyl)-5-methyl-1,2-dihydroxy-benzol und 11,0 g (0,504 Mol) Dibromessigsäure die 5-(2,6-Dichlorbenzoyl)-6-methyl-1,3-benzodioxol-2-carbonsäure als farblose Kristalle vom Smp. 118-122°.

Das Ausgangsketon wird wie folgt hergestellt:

a) Durch Umsetzung von 9,5 g (0,05 Mol) 2,6-Dichlorbenzoesäure und 7,5 g (0,05 Mol) 1,2-Dimethoxy-4-methylbenzol analog Beispiel 2a) erhält man das (2,6-Dichlorphenyl)-(3,4-dimethoxy-6-methyl-phenyl)-methanon als farblose Kristalle vom Smp. 101-103°.

b) Analog Beispiel 2b) erhält man aus dem Produkt von a) durch Aetherspaltung, das (2,6-Dichlorphenyl)-(3,4-dihydroxy-6-methylphenyl)-methanon als sandfarbene Kristalle vom Smp. 206-207°.

Beispiel 4: Analog Beispiel 2 erhält man durch Umsetzung von 23,8 g (0,0925 Mol (3,4-Dihydroxy-6-methylphenyl)-(2,6-dimethylphenyl)-methanon mit 23,4 g (0,107 Mol) Dibromessigsäure die 5-(2,6-Dimethylbenzoyl)-6-methyl-1,3-benzodioxol-2-carbonsäure als farblose Kristalle vom Smp. 129-131° (aus Toluol).

Das Ausgangsketon wird wie folgt hergestellt:

a) Analog Beispiel 2a) erhält man unter Verwendung von 2,6-Dimethylbenzoesäure und 1,2-Dimethoxy-4-methylbenzol das (3,4-Dimethoxy-6-methylphenyl)-(2,6-dimethylphenyl)-methanon als sandfarbene Kristalle vom Smp. 95-97°.

b) Analog Beispiel 2b) erhält man durch Aetherspaltung von 28 g (0,098 Mol) des Produktes von a) das (3,4-Dihydroxy-6-methylphenyl)-(2,6-dimethylphenyl)-methanon als farblose Kristalle vom Smp. 193-195° (aus Toluol).

Beispiel 5: Durch Umsetzung von 20,5 g (0,084 Mol) 4-(2-Methylbenzoyl)-5-methyl-1,2-dihydroxy-benzol und 21,3 g (0,0977 Mol) Dibromessigsäure analog Beispiel 2 und anschliessende Salzbildung mit 2-Amino-2,2-bis-(hydroxymethyl)-äthanol erhält man das [1,1-Bis-(hydroxymethyl)-2-hydroxyäthyl]-ammoniumsalz der 5-(2-Methyl-benzoyl)-6-methyl-1,3-benzodioxol-2-carbonsäure als farblose Kristalle vom Smp. 160-162°.

Das Ausgangsketon wird wie folgt hergestellt:

a) Analog Beispiel 2a) erhält man die Umsetzung von 13,6 g (0,1 Mol) 2-Methylbenzoesäure mit 15,2 g (0,1 Mol) 1,2-Dimethoxy-4-methylbenzol das (3,4-Dimethoxy-6-methylphenyl)-(2-methylphenyl)-methanon als braunes harzartiges Produkt.

b) Analog Beispiel 2b) erhält man durch Aetherspaltung aus dem Produkt von a) das (3,4-Dihydroxy-6-methylphenyl)-(2-methylphenyl)-methanon als sandfarbene Kristalle vom Smp. 149-150°.

Beispiel 6: Analog Beispiel 2 erhält man durch Umsetzung von 17,3 g (0,075 Mol (3,4-Dihydroxy-6-methylphenyl)-(3-pyridyl)-methanon mit 16,5 g (0,075 Mol) Dibromessigsäure die 5-Methyl-6-nicotinoyl-1,3-benzodioxol-2-carbonsäure als sandfarbene Kristalle vom Smp. 230-232° (aus Dimethylsulfoxid/Wasser).

a) Das Ausgangsketon wird wie folgt hergestellt:
61,5 g (0,5 Mol) Nicotinsäure werden mit 250 ml Thionylchlorid übergossen und das Gemisch 2 Stunden unter Rückfluss gekocht. Anschliessend wird das Thionylchlorid unter Normaldruck abdestilliert und zum Rückstand 500 ml Schwefelkohlenstoff zugegeben.

Unter kräftigem Rühren fügt man innerhalb 15 Minuten 250 g Aluminiumchlorid zu und rührt das Gemisch 2 Stunden bei Raumtemperatur. Nun werden innerhalb 30 Minuten 62 g (0,5 Mol) 4-Methyl-1,2-dihydroxy-benzol in Portionen zugegeben. Man rührt die dicke Suspension, bis kein Chlorwasserstoff mehr entweicht, dann destilliert man den Schwefelkohlenstoff bei einer Badtemperatur von 90° unter Normaldruck ab, setzt den Rückstand bei gleicher Temperatur für eine Stunde unter ein Vakuum von ca. 14 mbar, stellt anschliessend mit Stickstoff Normaldruck her und erhitzt die Reaktionsmasse noch 8 Stunden auf 100°.

Zum Aufarbeiten wird abgekühlt, die harte schaumartige Masse zerkleinert und auf Wasser ausgetragen. Das Reaktionsprodukt wird mittels Aethylacetat extrahiert und die Aethylacetatlösung eingeengt, wobei das (3,4-Dihydroxy-6-methyl)-(3-pyridyl)-methanon in sandfarbenen Kristallen anfällt, Smp. 220-223°.

Beispiel 7: Durch Umsetzung von 26,3 g (0,885 Mol) (2-Brom-4,5-dihydroxyphenyl)-phenylmethanon und 38,6 g (0,177 Mol) Dibromessigsäure analog Beispiel 2 und anschliessende Salzbildung mit Natronlauge erhält man das Natriumsalz der 5-Benzoyl-6-brom-1,3-benzodioxol-2-carbonsäure als farblose Kristalle, die unter Zersetzung oberhalb 150° schmelzen.

Das Ausgangsketon wird wie folgt hergestellt:

a) Durch Umsetzung von Benzoesäure mit 1-Brom-3,4-dimethoxybenzol analog Beispiel 2a) erhält man das (2-Brom-4,5-dimethoxyphenyl)-phenylmethanon vom Smp. 71-72°.

b) Analog Beispiel 2b) erhält man aus dem Produkt von a) durch Aetherspaltung das (2-Brom-4,5-dihydroxyphenyl)-phenylmethanon als braunes Oel.

Beispiel 8: Analog zu Beispiel 2 erhält man aus (2-Chlor-4,5-dihydroxyphenyl)-(3-chlorphenyl)-methanon und Dibromessigsäure 5-(3-Chlorbenzoyl)-6-chlor-1,3-benzodioxol-2-carbonsäure, Rf (Kieselgel) = 0.80 (System: Toluol/Dioxan/Eisessig/Ameisensäure 10:6:1:1). Ihr Aethylester schmilzt bei 98-98.5°.

Beispiel 9: Analog zu Beispiel 2 erhält man aus (2-Aethyl-4,5-dihydroxyphenyl)-phenylmethanon und Dibromessigsäure 5-Aethyl-6-benzoyl-1,3-benzodioxol-2-carbonsäure, welche analog Beispiel 7 in ihr Natriumsalz überführt wird, das bei 213-217° schmilzt und 1/3 Mol Kristallwasser enthält.

Beispiel 10: Um 1000 Kapseln mit je 100 mg Wirkstoffgehalt herzustellen, mischt man 100 g 5-(2-Chlorbenzoyl)-6-methyl-1,3-benzodioxol-2-carbonsäure mit 173,0 g Lactose, befeuchtet die Mischung gleichmässig mit einer wässrigen Lösung von 2,0 g Gelatine und granuliert sie durch ein geeignetes Sieb (z.B. Sieb III nach Ph. Helv. V.). Das Granulat mischt man mit 10,0 g getrockneter Maisstärke und 15,0 g Talk und füllt es gleichmässig in 1000 Hartgelatinekapseln der Grösse 1.

Anstelle der obigen kann man auch eine andere der in den vorangehenden Beispielen beschriebenen Verbindungen als Wirkstoff verwenden.

## Patentansprüche für die Vertragsstaaten: BE, DE, FR, IT, LU, NL, SE, CH, GB

1. Benzodioxolderivate der allgemeinen Formel I

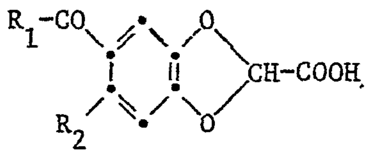

(I) ,

in welcher $R_1$ Pyridyl, Phenyl, 3-Halogenphenyl oder Phenyl, das in 2- und/oder 6-Stellung unabhängig voneinander durch Niederalkyl oder Halogen (di)substituiert ist, bedeutet, und $R_2$ Niederalkyl oder Halogen ist, als Racemate oder optische Antipoden, sowie Salze von solchen Verbindungen mit Basen, sowie Säureadditionssalze von solchen Verbindungen der allgemeinen Formel I, deren Rest $R_1$ basischen Charakter aufweist.

2. Verbindungen der allgemeinen Formel I gemäss Anspruch 1, in denen $R_1$ 3-Pyridyl, Phenyl, 3-Halogenphenyl, insbesondere 3-Chlorphenyl, oder Phenyl, das in 2- und/oder 6 Stellung unabhängig voneinander durch Niederalkyl, insbesondere Methyl, oder Halogen, insbesondere Chlor oder Brom, (di)substituiert ist, bedeutet und $R_2$ Niederalkyl, insbesondere Methyl oder Aethyl, oder Halogen, insbesondere Chlor oder Brom bedeutet, als Racemate oder optische Antipoden, sowie Salze von solchen Verbindungen mit Basen, sowie Säureadditionssalze von solchen Verbindungen der allgemeinen Formel I, deren Rest $R_1$ basischen Charakter aufweist.

3. Die 5-(2-Chlorbenzoyl)-6-methyl-1,3-benzodioxol-2-carbonsäure und ihre Salze mit Basen.

4. Die 5-(2-Brombenzoyl)-6-methyl-1,3-benzodioxol-2-carbonsäure und ihre Salze mit Basen.

5. Die 5-(2,6-Dichlorbenzoyl)-6-methyl-1,3-benzodioxol-2-carbonsäure und ihre Salze mit Basen.

6. Die 5-(2,6-Dimethylbenzoyl)-6-methyl-1,3-benzodioxol-2-carbonsäure und ihre Salze mit Basen.

7. Die 5-(2-Methylbenzoyl)-6-methyl-1,3-benzodioxol-2-carbonsäure und ihre Salze mit Basen.

8. Die 5-Methyl-6-nicotinoyl-1,3-benzodioxol-2-carbonsäure und ihre Salze mit Basen, sowie ihre Säureadditionssalze.

9. Die 5-Benzoyl-6-brom-1,3-benzodioxol-2-carbonsäure und ihre Salze mit Basen.

10. Die 5-(3-Chlorbenzoyl)-6-chlor-1,3-benzodioxol-2-carbonsäure und ihre Salze mit Basen.

11. Die 5-Aethyl-6-benzoyl-1,3-benzodioxol-2-carbonsäure und ihre Salze mit Basen.

12. Pharmazeutische Präparate, enthaltend eine Verbindung gemäss einem der Ansprüche 1 bis 9 oder ein pharmazeutisch annehmbares Salz einer solchen Verbindung.

13. Pharmazeutische Präparate, enthaltend eine Verbindung gemäss einem der Ansprüche 10 und 11 oder ein pharmazeutisch annehmbares Salz einer solchen Verbindung.

14. Verbindungen gemäss einem der Ansprüche 1 bis 9 und pharmazeutisch annehmbare Salze dieser Verbindungen zur Anwendung in einem Verfahren zur prophylaktischen und/oder therapeutischen Behandlung des tierischen oder menschlichen Körpers.

15. Verbindungen gemäss einem der Ansprüche 10 und 11 und pharmazeutisch annehmbare Salze dieser Verbindungen zur Anwendung in einem Verfahren zur prophylaktischen und/oder therapeutischen Behandlung des tierischen oder menschlichen Körpers.

16. Verbindungen gemäss einem der Ansprüche 1 bis 9 und pharmazeutisch annehmbare Salze dieser Verbindungen als Diuretika mit urikosurischer Zusatzwirkung.

17. Verbindungen gemäss einem der Ansprüche 10 und 11 und pharmazeutisch annehmbare Salze dieser Verbindungen als Diuretika mit urikusorischer Zusatzwirkung.

18. Verwendung von Verbindungen gemäss einem der Ansprüche 1 bis 9 oder von pharmazeutisch annehmbaren Salzen dieser Verbindungen zur Herstellung von Arzneimitteln auf nicht-chemischem Wege.

19. Verwendung von Verbindungen gemäss einem der Ansprüche 10 und 11 oder von pharmazeutisch annehmbaren Salzen dieser Verbindungen zur Herstellung von Arzneimitteln auf nicht-chemischem Wege.

20. Verfahren zur Herstellung von Benzodioxolderivaten der allgemeinen Formel I gemäss Anspruch 1, in welcher $R_1$ und $R_2$ die dort definierte Bedeutung haben, und von Salzen derselben, dadurch gekennzeichnet, dass man

a) eine Verbindung der allgemeinen Formel II

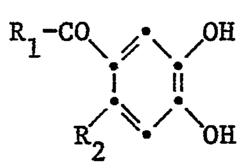

(II),

in welcher $R_1$ und $R_2$ die unter der Formel I angegebene Bedeutung haben, oder ein Salz davon mit einer Verbindung der allgemeinen

Formel III

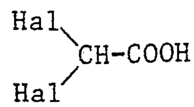

(III) ,

in welcher Hal Halogen bedeutet, oder einem Salz einer solchen Verbindung umsetzt, oder

b) in einer Verbindung der allgemeinen Formel IV

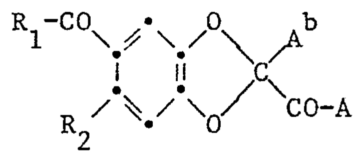

(IV),

in welcher $A^b$ Carboxy, Niederalkoxycarbonyl oder Acetyl bedeutet, A Hydroxy ist, und $R_1$ und $R_2$ die unter der Formel I angegebene Bedeutung haben, den Rest $A^b$ durch Wasserstoff ersetzt, oder

c) zur Herstellung einer Verbindung der allgemeinen Formel I, in welcher $R_1$ und $R_2$ die unter der Formel I angegebene Bedeutung haben, oder eines Salzes dieser Verbindung, in einer Verbindung der allgemeinen Formel VII

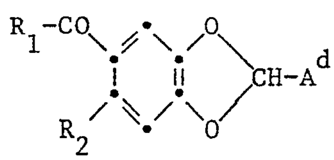

(VII) ,

in welcher $A^d$ eine in die Carboxygruppe überführbare Gruppe bedeutet, und $R_1$ und $R_2$ die unter der Formel I angegebene Bedeutung haben, die Gruppe $A^d$ in die Carboxygruppe in freier oder Salzform überführt, und gewünschtenfalls eine erhaltene Verbindung der allgemeinen Formel I in an sich bekannter Weise in eine andere Verbindung der allgemeinen Formel I überführt, und/oder eine als Racemat erhaltene Verbindung der allgemeinen Formel I in die optischen Antipoden zerlegt, und/oder eine erhaltene Verbindung der allgemeinen Formel I in ein Salz mit einer Base überführt oder eine

solche Verbindung aus einem erhaltenen Salz freisetzt, oder eine erhaltene Verbindung der allgemeinen Formel I von basischem Charakter in ein Säureadditionssalz überführt oder eine solche Verbindung aus einem erhaltenen Salz freisetzt.

21. Die nach dem Verfahren des Anspruchs 20 erhältlichen Verbindungen der im Anspruch 1 angegebenen allgemeinen Formel I, in welcher $R_1$ und $R_2$ die dort definierte Bedeutung haben, und Salze dieser Verbindungen.

FO 7.4 BL/mg*/hc*

## Patentansprüche für den Vertragsstaat AT

1. Verfahren zur Herstellung von Benzodioxolderivaten der allgemeinen Formel I

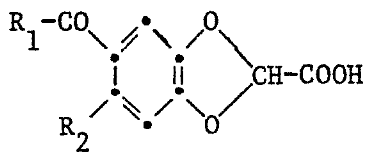

(I) ,

in welcher $R_1$ Pyridyl, Phenyl, 3-Halogenphenyl oder Phenyl, das in 2- und/oder 6-Stellung unabhängig voneinander durch Niederalkyl oder Halogen (di)substituiert ist, bedeutet, und $R_2$ Niederalkyl oder Halogen ist, und von Salzen derselben, dadurch gekennzeichnet, dass man

a) eine Verbindung der allgemeinen Formel II

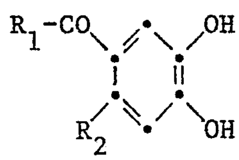

(II),

in welcher $R_1$ und $R_2$ die unter der Formel I angegebene Bedeutung haben, oder ein Salz davon mit einer Verbindung der allgemeinen Formel III

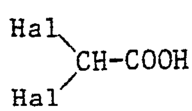

(III) ,

in welcher Hal Halogen bedeutet, oder einem Salz einer solchen Verbindung umsetzt, oder

b) in einer Verbindung der allgemeinen Formel IV

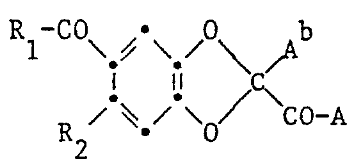

(IV),

in welcher $A^b$ Carboxy, Niederalkoxycarbonyl oder Acetyl bedeutet, A Hydroxy ist, und $R_1$ und $R_2$ die unter der Formel I angegebene Bedeutung haben, den Rest $A^b$ durch Wasserstoff ersetzt, oder

c) zur Herstellung einer Verbindung der allgemeinen Formel I, in welcher $R_1$ und $R_2$ die unter der Formel I angegebene Bedeutung haben, oder eines Salzes dieser Verbindung, in einer Verbindung der allgemeinen Formel VII

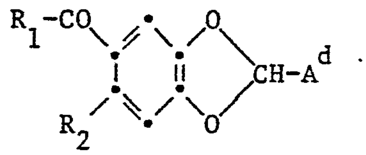

(VII) ,

in welcher $A^d$ eine in die Carboxygruppe überführbare Gruppe bedeutet, und $R_1$ und $R_2$ die unter der Formel I angegebene Bedeutung haben, die Gruppe $A^d$ in die Carboxygruppe in freier oder Salzform überführt, und gewünschtenfalls eine erhaltene Verbindung der allgemeinen Formel I in an sich bekannter Weise in eine andere Verbindung der allgemeinen Formel I überführt, und/oder eine als Racemat erhaltene Verbindung der allgemeinen Formel I in die optischen Antipoden zerlegt, und/oder eine erhaltene Verbindung der allgemeinen Formel I in ein Salz mit einer Base überführt oder eine solche Verbindung aus einem erhaltenen Salz freisetzt, oder eine erhaltene Verbindung der allgemeinen Formel I von basischem Charakter in ein Säureadditionssalz überführt oder eine solche Verbindung aus einem erhaltenen Salz freisetzt.

2. Verfahren nach Anspruch 1, dadurch gekennzeichnet, dass man Verbindungen der allgemeinen Formel I gemäss Anspruch 1, in denen $R_1$ 3-Pyridyl, Phenyl, 3-Halogenphenyl, insbesondere 3-Chlorphenyl, oder Phenyl, das in 2- und/oder 6-Stellung unabhängig voneinander durch Niederalkyl, insbesondere Methyl, oder Halogen, insbesondere Chlor oder Brom, (di)substituiert ist, bedeutet und $R_2$ Niederalkyl, inbesondere Methyl oder Aethyl, oder Halogen, insbesondere Chlor oder Brom bedeutet, herstellt.

3. Verfahen nach Anspruch 1, dadurch gekennzeichnet, dass man 5-(2-Chlorbenzoyl)-6-methyl-1,3-benzodioxol-2-carbonsäure und ihre Salze mit Basen herstellt.

4. Verfahren nach Anspruch 1, dadurch gekennzeichnet, dass man 5-(2-Brombenzoyl)-6-methyl-1,3-benzodioxol-2-carbonsäure und ihre Salze mit Basen herstellt.

5. Verfahren nach Anspruch 1, dadurch gekennzeichnet, dass man 5-(2,6-Dichlorbenzoyl)-6-methyl-1,3-benzodioxol-2-carbonsäure und ihre Salze mit Basen herstellt.

6. Verfahren anch Anspruch 1, dadurch gekennzeichnet, dass man 5-(2,6-Dimethylbenzoyl)-6-methyl-1,3-benzodioxol-2-carbonsäure und ihre Salze mit Basen herstellt.

7. Verfahren nach Anspruch 1, dadurch gekennzeichnet, dass man 5-(2-Methyl-benzoyl)-6-methyl-1,3-benzodioxol-2-carbonsäure und ihre Salze mit Basen herstellt.

8. Verfahren nach Anspruch 1, dadurch gekennzeichnet, dass man 5-Methyl-6-nicotinoyl-1,3-benzodioxol-2-carbonsäure und ihre Salze mit Basen, sowie ihre Säureadditionssalze herstellt.

9. Verfahren nach Anspruch 1, dadurch gekennzeichnet, dass man 5-Benzoyl-6-brom-1,3-benzodioxol-2-carbonsäure und ihre Salze mit Basen herstellt.

10. Verfahren nach Anspruch 1, dadurch gekennzeichnet, dass man 5-(3-Chlorbenzoyl)-6-chlor-1,3-benzodioxol-2-carbonsäure und ihre Salze mit Basen herstellt.

11. Verfahren nach Anspruch 1, dadurch gekennzeichnet, dass man 5-Aethyl-6-benzoyl-1,3-benzodioxol-2-carbonsäure und ihre Salze mit Basen herstellt.

12. Verfahren zur Herstellung eines pharmazeutischen Präparates, gekennzeichnet durch die Verarbeitung eines erfindungsgemässen Wirkstoffes nach einem der Ansprüche 1 bis 9 mit einem pharmazeutischen Trägermaterial.

13. Verfahren zur Herstellung eines pharmazeutischen Präparates, gekennzeichnet durch die Verarbeitung eines erfindungsgemässen Wirkstoffes nach einem der Ansprüche 10 und 11 mit einem pharmazeutischen Trägermaterial.

0107623

Europäisches Patentamt

# EUROPÄISCHER RECHERCHENBERICHT

Nummer der Anmeldung

EP 83 81 0480

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-2 426 505 (RICHARDSON-MERRELL)<br>* Seiten 1-13, 18-30 * | 1,12, 13,17, 20 | C 07 D 317/46<br>C 07 D 405/06<br>A 61 K 31/335<br>A 61 K 31/44 |
| P,X | EP-A-0 064 027 (CIBA-GEIGY)<br>* Insgesamt * | 1,2,9, 12,13, 16-21 | |
| | | | RECHERCHIERTE SACHGEBIETE (Int. Cl. 3) |
| | | | C 07 D 317/00 |

Der vorliegende Recherchenbericht wurde für alle Patentansprüche erstellt.

| Recherchenort | Abschlußdatum der Recherche | Prüfer |
|---|---|---|
| DEN HAAG | 19-01-1984 | FRANCOIS J.C.L. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82